(19) Europäisches Patentamt
European Patent Office
Office européen des brevets

(11) Veröffentlichungsnummer: **0 216 243**
**A2**

(12) # EUROPÄISCHE PATENTANMELDUNG

(21) Anmeldenummer: 86112459.2

(22) Anmeldetag: 09.09.86

(51) Int. Cl.⁴: **C 07 D 209/48**
**A 01 N 43/38, A 01 N 47/12**

(30) Priorität: 19.09.85 DE 3533440

(43) Veröffentlichungstag der Anmeldung:
01.04.87 Patentblatt 87/14

(84) Benannte Vertragsstaaten:
AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Liebl, Rainer, Dr.
An der Weinleite 5b
D-8901 Todtenweis(DE)

(72) Erfinder: Handte, Reinhard, Dr.
Theilweg 23
D-8901 Gablingen(DE)

(72) Erfinder: Mildenberger, Hilmar, Dr.
Fasanenstrasse 24
D-6233 Kelkheim (Taunus)(DE)

(72) Erfinder: Bauer, Klaus, Dr.
Kolpingstrasse 7
D-6054 Rodgau(DE)

(72) Erfinder: Bieringer, Hermann, Dr.
Eichenweg 26
D-6239 Eppstein/Taunus(DE)

(54) **N-Substituierte 3,4,5,6-Tetrahydrophthalimide, Verfahren zu ihrer Herstellung sowie ihre Verwendung im Pflanzenschutz.**

(57) Die Verbindungen der Formel I

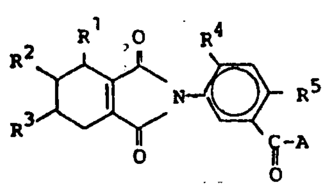

(I),

worin
$R^1, R^2, R^3$ = H oder Alkyl, $R^4$ = Wasserstoff, F, Cl, Br, Alkyl oder Alkoxy, $R^5$ = F, Cl, Br, (subst.)Alkyl, (subst.)Alkoxy, (subst.)Phenoxy, (subst.)Phenylthio, Alkylthio, Alkylsulfonyl, (subst.)Phenylsulfonyl; A= einen Rest der Formeln

$-Y-[C(R_6)_2]_n-X-COYR^7$,

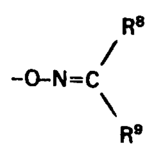

,

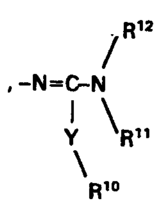

,

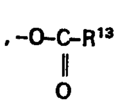

oder

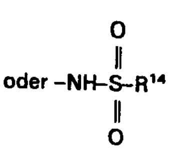

,

bedeuten, besitzen vorteilhafte herbizide Eigenschaften gegenüber mono- und dikotylen Schadpflanzen.

EP 0 216 243 A2

N-Substituierte 3,4,5,6-Tetrahydrophthalimide, Verfahren zu ihrer Herstellung sowie ihre Verwendung im Pflanzenschutz

Es ist bekannt, daß bestimmte Tetrahydrophthalimide herbizide Eigenschaften aufweisen (z.B. US-PS 3 878 224, EP-A-049 508, DE-OS 31 09 035).

Es wurden nun neue N-Aryl-3,4,5,6-tetrahydrophthalimide gefunden, die vorteilhafte herbizide Eigenschaften aufweisen und eine überraschend gute Verträglichkeit gegenüber Kulturpflanzen zeigen.

Gegenstand der vorliegenden Erfindung sind daher die Verbindungen der Formel I

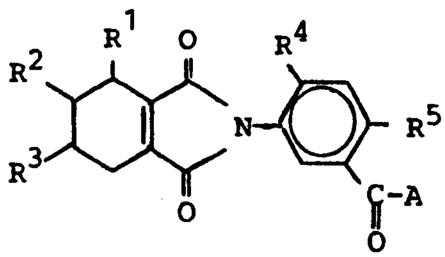 (I),

worin

$R^1$, $R^2$, $R^3$ = unabhängig voneinander Wasserstoff oder $(C_1-C_4)$Alkyl,

$R^4$ = Wasserstoff, F, Cl, Br, $(C_1-C_4)$Alkyl oder $(C_1-C_4)$Alkoxy,

$R^5$ = F, Cl, Br, $(C_1-C_4)$Alkyl, $(C_1-C_4)$Alkoxy, wobei beide letztgenannten Reste bis zu 3-fach durch Halogen substituiert sein können; Phenoxy, Phenylthio wobei in den beiden letztgenannten Resten der Phenylkern bis zu 3-fach durch Halogen, $(C_1-C_4)$-Alkyl, $(C_1-C_4)$Alkoxy oder $CF_3$ substituiert sein kann, $(C_1-C_4)$Alkylthio, $(C_1-C_4)$Alkylsulfonyl, Phenylsulfonyl, wobei der Phenylring bis zu 3-fach substituiert sein kann durch Halogen, $(C_1-C_4)$Alkyl, $(C_1-C_4)$Alkoxy oder $CF_3$;

A = einen Rest der Formel 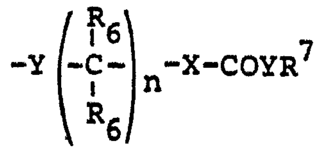

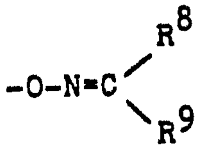 , 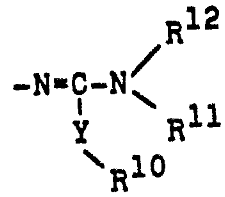 , 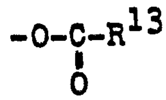

oder 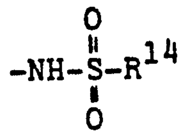 ,

$R^6$ = Wasserstoff, $(C_1-C_4)$Alkyl, $CCl_3$, Phenyl, das bis zu 3-fach durch $(C_1-C_4)$Alkyl $(C_1-C_4)$Alkoxy, Halogen oder $CF_3$ substituiert sein kann; oder $(C_1-C_4)$Alkoxycarbonyl$(C_1-C_4)$-alkyl

$R^7$ = H, $(C_1-C_{12})$Alkyl, $(C_1-C_4)$Alkoxy$(C_1-C_4)$ alkyl, $(C_1-C_4)$Alkoxycarbonyl$(C_1-C_4)$alkyl, $(C_2-C_4)$-Alkenyl, $(C_3-C_4)$Alkinyl, $(C_3-C_7)$Cycloalkyl, Halogen$(C_1-C_4)$alkyl mit ein bis 3 Halogenatomen, Phenyl oder Benzyl, die beide bis zu 3-fach am Phenylring durch Halogen, $(C_1-C_4)$-Alkyl, $(C_1-C_4)$Alkoxy oder $CF_3$ substituiert sein können,

X = eine direkte Bindung oder einen Rest der Formeln

O-

$-CH_2-$, $-O-CH_2-$ oder $-CH(C_1-C_4Alkyl)$, wobei die beiden letztgenannten Reste jeweils am C-Atom an die Gruppe $-COYR^7$ gebunden sind,

Y = O, S, $NR^{15}$,

$R^8$, $R^9$ = $(C_1-C_8)$Alkyl, $(C_3-C_7)$Cycloalkyl, $(C_1-C_4)$-Alkoxycarbonyl, Phenyl, Benzyl, die beide im Phenylring 1- bis 3fach durch $(C_1-C_4)$-Alkyl, $(C_1-C_4)$Alkoxy, das halogeniert sein kann, $(C_1-C_4)$Alkylthio, $(C_1-C_4$-Alkoxy)-carbonyl, $NO_2$, Halogen, CN oder $CF_3$, substituiert sein können, oder

$R^8$, $R^9$ zusammen mit dem sie verbindenden C-Atom einen 3- bis 7gliedrigen gesättigten oder einfach ungesättigten aliphatischen Ring bilden, der 1- bis 3fach durch $(C_1-C_4)$Alkyl, $(C_1-C_4)$Alkoxy, Halogen oder $(C_1-C_4$-Alkoxy)carbonyl substituiert sein kann,

$R^{10}$ = $(C_1-C_4)$Alkyl oder Benzyl,

$R^{11}$, $R^{12}$ = Wasserstoff oder die Gruppe $=CH-N(C_1-C_4alkyl)_2$

$R^{13}$ = $(C_1-C_8)$Alkyl oder Phenyl, das 1- bis 3fach durch Halogen, $(C_1-C_4)$-Alkyl, $(C_1-C_4)$Alkoxy oder $(C_1-C_4$-Alkoxy)carbonyl substituiert sein kann,

$R^{14}$ = Hydroxy oder $(C_1-C_4)$Alkyl,

$R^{15}$ = H, $(C_1-C_4)$Alkyl oder Phenyl und

n = 1, 2 oder 3

bedeuten.

Von den Verbindungen der Formel I sind solche bevorzugt, worin
$R^1$, $R^2$, $R^3$ = Wasserstoff, $R^4$ = H, F oder Cl, $R^5$= F,Cl, Br, $(C_1-C_4)$Alkyl oder $(C_1-C_4)$Alkoxy, A = einen Rest der Formel

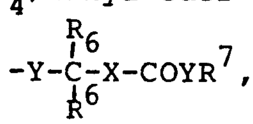, $-O-N=CR^8R^9$ oder 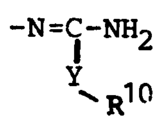

wobei bevorzugt einer der Reste $R^6$ Wasserstoff und der andere $(C_1-C_4)$Alkyl oder $CCl_3$ oder beide Reste $R^6$ $(C_1-C_4)$-Alkyl bedeuten; $R^7$= $(C_1-C_4)$Alkyl, $(C_3-C_4)$Alkinyl oder $(C_1-C_4)$Alkoxy$(C_1-C_4)$alkyl; $R^8$,$R^9$= $(C_1-C_4)$Alkyl, $(C_1-C_4)$-Alkoxycarbonyl oder zusammen mit dem sie verbindenden C-Atom einen $(C_5-C_6)$Cycloalkylring bedeuten.

Halogeniertes $(C_1-C_4)$Alkyl oder halogeniertes $(C_1-C_4)$Alkoxy enthält insbesondere ein bis drei F, Cl oder Br-Atome. Bevorzugt sind $CF_3$, $C_2F_5$, $OCF_3$, $OCH_2CF_3$ oder $OC_2F_5$.

Im Falle von $R^7$ = H oder $R^{14}$ = OH können aus den Verbindungen der Formel I auch Salze hergestellt werden. Diese werden ebenfalls von vorliegender Erfindung umfaßt.

Ferner können die Verbindungen der Formel I zum Teil (z.B. für A= $Y-CH(R_6)_2-X-CO_2R^7$) in Form von Enantiomeren, Diasteromeren oder deren Gemische vorliegen. Die Erfindung umfaßt alle diese Stereoisomeren.

Ein weiterer Gegenstand der Erfindung sind Verfahren zur Herstellung der Verbindungen der allgemeinen Formel I, dadurch gekennzeichnet, daß man Verbindungen der Formel II

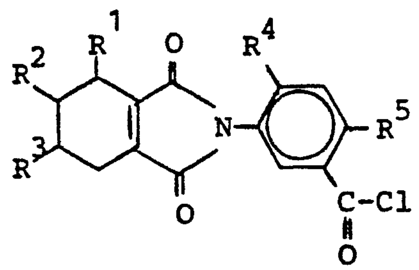

(II)

in Gegenwart einer Base mit einer Verbindung der Formel H-A, worin A die obengenannte Bedeutung besitzt, umsetzt.

Als Basen können anorganische Basen, wie Alkalihydroxyd oder Alkalicarbonat wie NaOH, $K_2CO_3$ oder organische Basen, beispielsweise tertiäre Stickstoffbasen wie Pyridin oder Triethylamin, verwendet werden. Man arbeitet zweckmäßigerweise in einem unter den Reaktionsbedingungen inerten organischen Lösungsmittel, wie Toluol, Methylenchlorid, Acetonitril, Dimethylformamid oder Diethylether. Die Reaktion kann auch im Zweiphasensystem Wasser/organisches Lösungsmittel wie z.B. Toluol, Methylenchlorid ausgeführt werden. Dabei wird die Komponente H-A im Zweiphasensystem Wasser/organisches Lösungsmittel gelöst und die Verbindung der Formel II gleichzeitig mit der Base zugetropft. Die Reaktionstemperaturen können zwischen 0° und 80°, vorzugsweise zwischen 10° und 50°C variieren.

Die Verbindungen der Formel II lassen sich durch Halogenierung der entsprechenden Säuren auf üblichem Wege mit einem Halogenierungsmittel beispielsweise mit Thionylchlorid,

Phosphortri- (oder -penta)chlorid gewinnen. Die entsprechenden Säuren sind bekannt (z.B. aus DE-OS 31 09 035) oder lassen sich analog den dort beschriebenen Verfahren aus den entsprechenden Tetrahydrophthalsäureanhydriden und den entsprechenden Anilinen herstellen. Die Umsetzung der Säuren zu den Säurechloriden der Formel II und die Folgereaktion zu den Verbindungen der Formel I lassen sich in einem Eintopf-Verfahren durchführen, wobei vor der Umsetzung von II zu I das Halogenierungsmittel aus dem Reaktionsmedium beispielsweise durch Destillation entfernt werden muß.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Beispielsweise können folgende Schadpflanzen bekämpft werden:
Schadgräser wie
Avena fatua, Alopecurus sp., Lolium sp., Setaria sp., Digitaria sp., Sorphum halepense, Echinochloa sp., Agropyron sp., Cynodon sp., Phalaris sp.,
dikotyle Pflanzen wie
Lamium sp., Veronica sp., Galium sp., Stellaria sp., Matricaria sp., Papaver sp., Centauria sp., Amaranthus sp., Galinsoga sp., Mercurialis sp., Sida sp., Abutilon sp., Ambrosia sp., Xanthium sp., Cirsium sp., Artemisia sp., Rumex sp., Convolvulus sp., Ipomea sp., Sinapis sp.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete

Wirkstoffe in der für die Herstellung von Düngemittelgranulaten üblichen Weise, gewünschtenfalls in Mischung mit Düngemitteln, granuliert werden.

In Spritzpulver beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Die Erfindung wird durch nachstehende Beispiele erläutert.

A. Formulierungsbeispiele

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## B. Chemische Beispiele

### Beispiel 1

**N-(2,4-Dichlor-5-methoxycarbonylmethoxycarbonyl-phenyl)-3,4,5,6-tetrahydrophthalimid**

35,9 g (0,1 mol) N-(2,4-Dichlor-5-chlorcarbonyl-phenyl)-3,4,5,6-tetrahydrophthalimid und 9,0 g (0,1 mol) Glykolsäuremethylester werden in 200 ml trockenem Toluol gelöst. Bei RT tropft man 7,9 g (0,1 mol) Pyridin zu und rührt 10 min bei RT. Anschließend läßt man noch 3 h bei 50 °C rühren, wäscht zweimal mit je 100 ml Wasser und trocknet über Natriumsulfat. Nach Abziehen des Lösungsmittels unter vermindertem Druck erhält man 39,8 g (97 % d. Th.) N-(2,4-Dichlor-5-methoxycarbonylmethoxycarbonyl-phenyl)-3,4,5,6-tetrahydrophthalimid in Form eines zähen, hellgelben Öles.

### Beispiel 2

**N-(2,4-Dichlor-5-isopropylidenaminoxycarbonyl-phenyl)-3,4,5,6-tetrahydrophthalimid**

Man löst 35,9 g (0,1 mol) N-(2,4-Dichlor-5-chlorcarbonyl-phenyl)-3,4,5,6-tetrahydrophthalimid und 7,3 g Acetonoxim in 200 ml Toluol und tropft bei RT 7,9 g Pyridin zu. Man rührt 1 h bei 20 °C und 2 h bei 50 °C, wäscht zweimal mit je 150 ml Wasser und trocknet die organische Phase über Natriumsulfat. Nach Abziehen des Lösungsmittels unter vermindertem Druck wird der verbleibende Rückstand mit 200 ml Ether/n-Hexan aufgekocht und der Niederschlag abgesaugt. Man erhält 39,2 g (99 % d. Th.) N-(2,4-Dichlor-5-isopropylidenaminoxycarbonyl-phenyl)-3,4,5,6-tetrahydrophthalimid in Form hellgelber Kristalle mit Schmp. 140-141 °C.

...

Beispiel 3

N-(2,4-Dichlor-5-pivaloyloxycarbonyl-phenyl)-3,4,5,6-tetrahydrophthalimid

Man löst 35,9 g (0,1 mol) N-(2,4-Dichlor-5-chlorcarbonyl-phenyl)-3,4,5,6-tetrahydrophthalimid und 10,2 g Pivalinsäure in 300 ml Toluol und tropft bei RT 7,9 g Pyridin zu. Man rührt 30 min bei RT und 6 h bei 40 °C. Das Reaktionsgemisch wird zweimal kurz mit je 100 ml Wasser gewaschen, die org. Phase über Natriumsulfat getrocknet und das Lösungsmittel unter vermindertem Druck abdestilliert. Man erhält 40,8 g (96 % d. Th.) N-(2,4-Dichlor-5-pivaloyloxycarbonyl-phenyl)-3,4,5,6-tetrahydrophthalimid in Form eines hellgelben Öles das langsam kristallisiert.

Beispiel 4

N-[2,4-Dichlor-5-(3,4,5,6-tetrahydrophthalimido)-benzoyl]-O-methyl-isoharnstoff

Man löst 13,5 g (0,11 mol) O-Methylisoharnstoff-sulfat in 100 ml Wasser und 200 ml Methylenchlorid. Bei 5 °C tropft man gleichzeitig 8,0 g (0,2 mol) Natronlauge in 20 ml Wasser und 35,9 g (0,1 mol) N-(2,4-Dichlor-5-chlorcarbonyl-phenyl)-3,4,5,6-tetrahydrophthalimid, gelöst in 100 ml Methylenchlorid, unter kräftigem Rühren zu. Man rührt noch 2 h bei 20 °C, trennt die organische Phase ab, wäscht mit 200 ml Wasser und trocknet über Natriumsulfat. Nach Abziehen des Lösungsmittels erhält man 38,1 g (96 % d. Th.) N-[2,4-Dichlor-5-(3,4,5,6-tetrahydrophthalimido)-benzoyl]-O-methyl-isoharnstoff in Form hellgelber Kristalle mit Schmp. 153-154 °C.

...

Beispiel 5

N-[2,4-Dichlor-5-(3,4,5,6-tetrahydrophthalimido)-benzoyl]-S-methyl-isothioharnstoff

Man löst 15,3 g (0,11 mol) S-Methylisothioharnstoff-sulfat in 100 ml Wasser und 150 ml Methylenchlorid. Bei 10 °C tropft man gleichzeitig 8,0 g (0,2 mol) Natronlauge in 20 ml Wasser und 35,9 g (0,1 mol) N-(2,4-Dichlor-5-chlorcarbonyl-phenyl)-3,4,5,6-tetrahydrophthalimid, gelöst in 100 ml Methylenchlorid, unter kräftigem Rühren zu. Man rührt noch 2 h bei 20 °C, trennt die organische Phase ab, wäscht mit 200 ml Wasser und trocknet über Natriumsulfat. Nach dem Abziehen des Lösungsmittels erhält man 37,9 g (92 % d. Th.) N-[2,4,-Dichlor-5-(3,4,5,6-tetrahydrophthalimido)-benzoyl]-S-methyl-isothioharnstoff in Form eines hellgelben Pulvers mit Schmp. 108-110 °C.

Beispiel 6

N-[2,4-Difluor-5-(1-ethoxycarbonyl-ethoxycarbonyl)-phenyl]-3,4,5,6-tetrahydrophthalimid

Man löst 32,6 g (0,1 mol) N-(5-Chlorcarbonyl-2,4-difluorphenyl)-3,4,5,6-tetrahydrophthalimid und 11,8 g Milchsäureethylester in 200 ml trockenem Toluol und tropft bei RT 10,1 g (0,1 mol) Triethylamin zu. Man rührt 1 h bei RT und 3 h bei 50 °C. Das Reaktionsgemisch wird zweimal mit je 100 ml Wasser gewaschen, über Natriumsulfat getrocknet und einrotiert. Man erhält 38,0 g (93 % d. Th.) N-[2,4-Difluor-5-(1-ethoxycarbonyl-ethoxycarbonyl)-phenyl]-3,4,5,6-tetrahydrophthalimid in Form eines zähen, hellgelben Öls.

Die in der nachfolgenden Tabelle 1 aufgeführten Verbindungen werden analog der obigen Beispiele hergestellt.

...

Tabelle 1



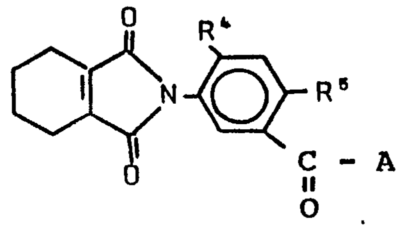

(I, $R^1 = R^2 = R^3 = H$)

| Bsp. | $R^4$ | $R^5$ | A | Fp [°C] |
|---|---|---|---|---|
| 7 | Cl | Cl | 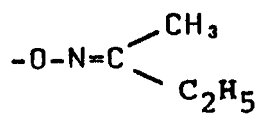 | gelbes Oel |
| 8 | Cl | Cl | 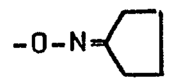 | 84-86 |
| 9 | F | F | 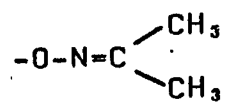 | 147-150 |
| 10 | F | F | 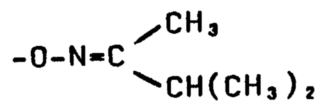 | |
| 11 | Cl | Cl | 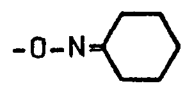 | |

...

Fortsetzung Tabelle 1

| Bsp. | $R^4$ | $R^5$ | A | Fp [°C] |
|---|---|---|---|---|
| 12 | F | F | $-O-CH_2-CO_2CH_3$ | gelbes Oel |
| 13 | Cl | Cl | $-O-CH(CCl_3)-O-CH(CH_3)-CO_2C_2H_5$ | " |
| 14 | H | Cl | $-O-CH(CH_3)-CO_2C_2H_5$ | gelbes Harz |
| 15 | H | Br | $-O-CH_2-CO_2CH_3$ | |
| 16 | F | F | $-O-C(=O)-CH(CH_3)_3$ | |
| 18 | F | F | $-N=C(OCH_3)-NH_2$ | gelbes Oel |
| 19 | F | F | $-N=C(SCH_3)-NH_2$ | " |
| 20 | F | F | $-O-CH(CCl_3)-O-CH(CH_3)-CO_2C_2H_5$ | |

0216243

Fortsetzung Tabelle 1

| Bsp. | $R^4$ | $R^5$ | A | Fp [°C] |
|---|---|---|---|---|
| 21 | Cl | Cl | $-S-CH_2-CO_2CH_3$ | gelbes Öl |
| 22 | Cl | Cl | $-S-CH(CH_3)-CO_2C_2H_5$ | " |
| 23 | F | F | $-S-CH(CH_3)-CO_2C_2H_5$ | " |
| 24 | H | Br | $-O-CH(CCl_3)-O-CH(CH_3)-CO_2C_2H_5$ | " |
| 25 | H | Br | $-O-N=C(CH_3)_2$ | 87 - 89 |
| 26 | H | F | $-O-N=C(CH_3)_2$ | 75 - 78 |
| 27 | H | Cl | $-O-N=C(CH_3)_2$ | |
| 28 | H | Cl | $-O-CH(CH_3)-CO_2C_4H_9$ | gelbes Öl |
| 29 | H | Cl | $-S-CH(CH_3)-CO_2C_2H_5$ | " |
| 30 | H | Br | $-S-CH(CH_3)-CO_2C_2H_5$ | " |

Fortsetzung Tabelle 1

| Bsp. | $R^4$ | $R^5$ | A | Fp [°C] |
|---|---|---|---|---|
| 31 | H | Br | $CH_3$<br>\|<br>$-O-CH-CO_2C_2H_5$ | hellbraunes Harz |
| 32 | H | Br | $CH_3$<br>\|<br>$-O-CH-CO_2CH(CH_3)_2$ | " |
| 33 | H | Br | $CH_3$<br>\|<br>$-O-CH-C-SC_2H_5$<br>‖<br>O | " |
| 34 | H | Br | $CCl_3$<br>\|<br>$-O-CH-CO_2C_2H_5$ | " |
| 35 | H | Br | $CH_3$<br>\|<br>$-O-CH-CO_2CH_2C\equiv CH$ | " |
| 36 | H | Br | $CH_3$<br>\|<br>$-O-CH-CO_2CH_2CH_2-OCH_3$ | " |
| 37 | H | Br | $C_4H_9$<br>\|<br>$-O-CH-CO_2C_2H_5$ | " |
| 38 | H | Br | $CH_3$<br>\|<br>$-O-CH-CH_2-CO_2C_2H_5$ | " |
| 39 | H | Br | $C_6H_5$<br>\|<br>$-O-CH-CO_2C_2H_5$ | " |
| 40 | H | Br | $CH_3$<br>\|<br>$-O-C-CO_2C_2H_5$<br>\|<br>$CH_3$ | " |
| 41 | H | Br | $CH_3$<br>\|<br>$-O-CH-CO_2$(Cyclo)$C_6H_{11}$ | " |
| 42 | H | Br | $CH_3$<br>\|<br>$-O-CH-CO_2C_8H_{17}$ | " |
| 43 | H | Br | 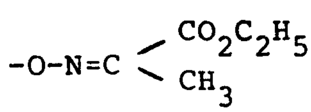 | " |
| 44 | H | Br | 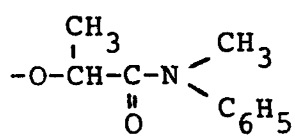 | " |
| 45 | H | Br | $CH_3$<br>\|<br>$-O-CH-CO_2C_{12}H_{25}$ | " |

Fortsetzung Tabelle 1

| Bsp. | $R^4$ | $R^5$ | A | Fp [°C] |
|---|---|---|---|---|
| 46 | H | Br | 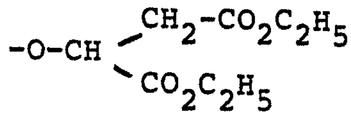 | hellbraunes Harz |
| 47 | H | Br | 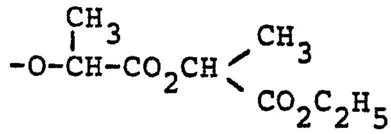 | " |
| 48 | H | Br | 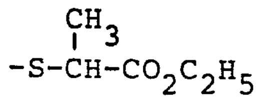 | " |
| 49 | H | Cl | 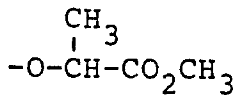 | " |
| 50 | H | Cl | 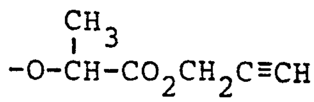 | " |
| 51 | H | Cl | 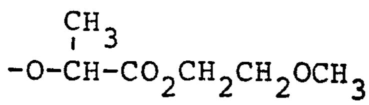 | " |
| 52 | H | Cl | 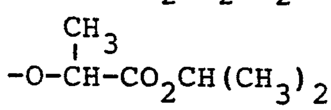 | " |
| 53 | H | Cl | 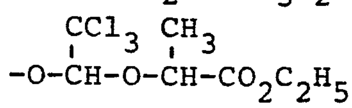 | " |
| 54 | H | Cl | 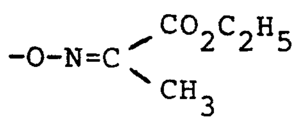 | " |
| 55 | H | Cl | 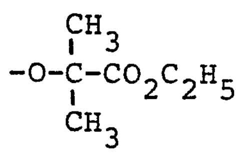 | " |
| 56 | H | Cl | 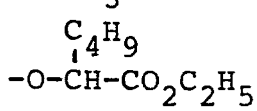 | " |
| 57 | H | Cl | 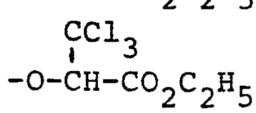 | 92 - 94 |
| 58 | H | Cl | 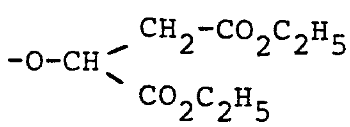 | hellbraunes Harz |
| 59 | H | Cl | 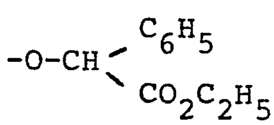 | " |

Fortsetzung Tabelle 1

| Bsp. | $R^4$ | $R^5$ | A | Fp [°C] |
|---|---|---|---|---|
| 60 | H | Cl | 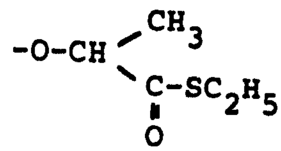 | hellbraunes Harz |
| 61 | H | Cl | 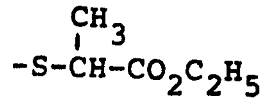 | " |
| 62 | H | Cl | 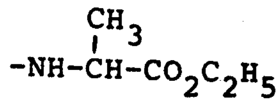 | " |
| 63 | H | F | 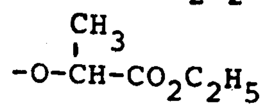 | " |
| 64 | H | F | 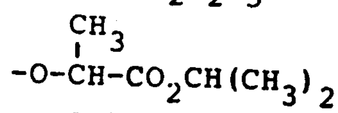 | " |
| 65 | H | F | 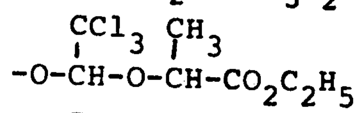 | " |
| 66 | H | F | 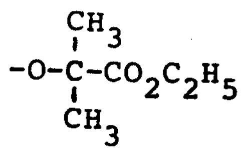 | " |
| 67 | H | F | 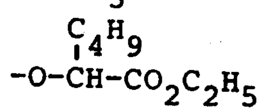 | " |
| 68 | H | F | 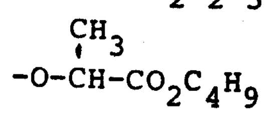 | " |
| 69 | H | F | 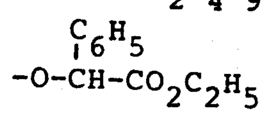 | " |
| 70 | H | F | 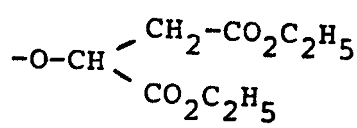 | " |
| 71 | H | F | 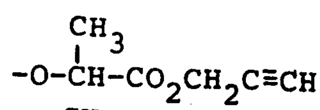 | " |
| 72 | H | F | 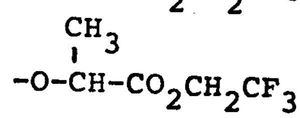 | " |
| 73 | H | Cl | 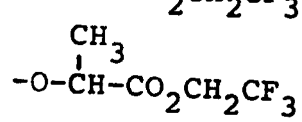 | " |
| 74 | H | Br | 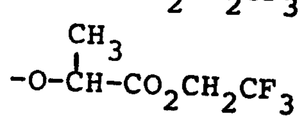 | " |

Fortsetzung Tabelle 1

| Bsp. | $R^4$ | $R^5$ | A | Fp [°C] |
|---|---|---|---|---|
| 75 | H | F | 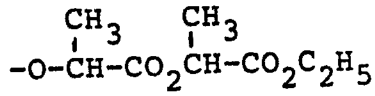 | hellbraunes Harz |
| 76 | H | F | 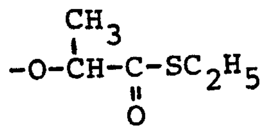 | " |
| 77 | H | F | 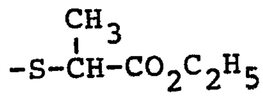 | " |
| 78 | H | F | 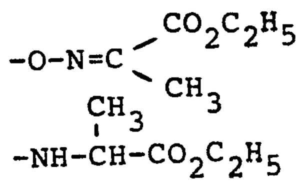 | " |
| 79 | H | F | | " |
| 80 | H | $-CH_3$ | 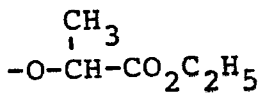 | " |
| 81 | H | $-OCH_3$ | 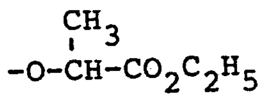 | " |
| 82 | H | $-OC_2H_5$ | 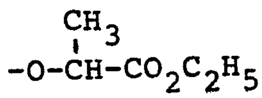 | " |
| 83 | H | $-OC_2H_5$ | 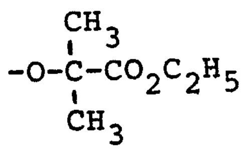 | " |
| 84 | H | $-SC_2H_5$ | 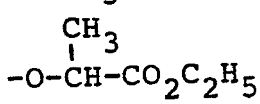 | " |
| 85 | H | $-SO_2C_2H_5$ | " | " |
| 86 | H | $-O-C_6H_5$ | " | " |
| 87 | H | $-O-C_6H_4-4-Cl$ | " | " |
| 88 | H | $-S-C_6H_5$ | " | " |
| 89 | H | $-S-C_6H_4-4-Cl$ | " | " |
| 90 | H | $-SO_2-C_6H_5$ | " | " |
| 91 | H | $-SO_2-C_6H_4-4-Cl$ | " | " |
| 92 | H | $-OCH_2CH_2-OCH_3$ | " | " |
| 93 | H | $-CF_3$ | " | " |
| 94 | F | Cl | " | " |
| 95 | F | Br | " | " |
| 96 | Cl | $-CH_3$ | " | |

Fortsetzung Tabelle 1

| Bsp. | $R^4$ | $R^5$ | A | Fp [°C] |
|---|---|---|---|---|
| 97 | Cl | $-OC_2H_5$ | $CH_3$<br>$-O-CH-CO_2C_2H_5$ | hellbraunes Harz |
| 98 | Cl | $-OCH_3$ | " | |
| 99 | F | $-OCH_3$ | " | |
| 100 | Br | $-OCH_3$ | " | |
| 101 | Br | $-CH_3$ | " | |
| 102 | H | F | $-N=C-NH_2$<br>$OCH_3$ | gelbes Harz |
| 103 | H | F | $-N=C-NH_2$<br>$OC_2H_5$ | " |
| 104 | H | F | $-N=C-NH_2$<br>$SCH_3$ | |
| 105 | H | F | $-N\ C-NH_2$<br>S<br>$CH_2C_6H_5$ | |
| 106 | H | Cl | $-N=C-NH_2$<br>$OCH_3$ | " |
| 107 | H | Cl | $-N=C-NH_2$<br>$OC_2H_5$ | " |
| 108 | H | Cl | $-N=C-NH_2$<br>$SCH_3$ | " |
| 109 | H | Cl | $-N=C-NH_2$<br>$SC_2H_5$ | |
| 110 | H | Br | $-N=C-NH_2$<br>$OCH_3$ | |
| 111 | H | Br | $-N=C-NH_2$<br>$SCH_3$ | |
| 112 | H | $CH_3$ | $-N=C-NH_2$<br>$OCH_3$ | |
| 113 | H | $OCH_3$ | " | |
| 114 | H | $OC_2H_5$ | " | |

Fortsetzung Tabelle 1

| Bsp. | $R^4$ | $R^5$ | A | Fp [°C] |
|---|---|---|---|---|
| 115 | H | $-O-C_6H_5$ | $-N=\underset{OCH_3}{\underset{\vert}{C}}-NH_2$ | |
| 116 | H | $-SC_2H_5$ | " | |
| 117 | H | $-SO_2C_2H_5$ | " | |
| 118 | H | $-S-C_6H_4-4-Cl$ | " | |
| 119 | H | $-SO_2-C_6H_5$ | " | |
| 120 | F | Cl | " | |
| 121 | F | Br | " | |
| 122 | F | $-CH_3$ | " | |
| 123 | Cl | $-CH_3$ | " | |
| 124 | F | $-OCH_3$ | " | |
| 125 | F | $-OC_2H_5$ | " | |
| 126 | Cl | $-OCH_3$ | " | |
| 127 | Cl | $-OC_2H_5$ | " | |

0216243

## C. Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60 - 80 %).

Die optische Bonitur der Pflanzen bzw. Auflaufschäden erfolgte nach dem Auflaufen der Versuchspflanzen nach einer Versuchszeit von 3 - 4 Wochen im Vergleich zu unbehandelten Kontrollen.
Die Boniturwerte zeigen, daß die erfindungsgemäßen Verbindungen eine gute herbizide Vorauflaufwirksamkeit gegen ein breites Spektrum von Ungräsern und Un-

...

kräutern besitzen, s. Tabelle 2.

Tabelle 2

| Bsp. Nr. | Dosis kg a.i./ha | herbizide Wirkung SIA | CRS | LOM | ECG |
|---|---|---|---|---|---|
| 2 | 2,5 | 5 | 3 | 5 | 5 |
| 4 | 2,5 | 4 | 5 | 4 | 4 |
| 5 | 2,5 | 3 | 5 | 2 | 4 |
| 6 | 2,5 | 2 | 5 | 1 | 4 |
| 14 | 2,5 | 2 | 5 | 3 | 4 |
| 18 | 2,5 | 5 | 5 | 5 | 4 |
| 24 | 2,5 | 4 | 5 | 5 | 5 |
| 28 | 2,5 | 2 | 5 | 4 | 5 |
| 29 | 2,5 | 2 | 5 | 4 | 5 |
| 38 | 2,5 | 1 | 4 | 2 | 3 |
| 40 | 2,5 | 3 | 5 | 3 | 4 |
| 54 | 2,5 | 5 | 5 | 5 | 4 |
| 57 | 2,5 | 2 | 5 | 1 | 5 |

## 2. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach Aussaat wurden die Versuchspflanzen im Dreiblattstadium behandelt.
Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1 °C, relative Luftfeuchte 60 - 80 %) die Wirkung der Präparate optisch im Vergleich zu unbehandelten Kontrollen bonitiert.

0216243

Die erfindungsgemäßen Verbindungen weisen auch im Nachauflauf eine gute herbizide Wirksamkeit gegen ein breites Spektrum wirtschaftlich wichtiger Ungräser und Unkräuter auf, s. Tabelle 3.

Tabelle 3

| Bsp.-Nr. | Dosis kg a.i./ha | herbizide Wirkung SIA | CRS | LOM | ECG |
|---|---|---|---|---|---|
| 1 | 2,5 | 5 | 5 | 2 | 2 |
| 6 | 2,5 | 4 | 5 | 1 | 3 |
| 13 | 2,5 | 5 | 5 | 2 | 2 |
| 14 | 2,5 | 5 | 5 | 4 | 4 |
| 18 | 2,5 | 4 | 5 | 2 | 3 |
| 22 | 2,5 | 5 | 5 | 2 | 3 |
| 24 | 2,5 | 5 | 5 | 4 | 4 |
| 25 | 2,5 | 4 | 5 | 2 | 2 |
| 28 | 2,5 | 5 | 5 | 4 | 4 |
| 29 | 2,5 | 5 | 5 | 4 | 4 |
| 30 | 2,5 | 4 | 5 | 2 | 3 |
| 31 | 2,5 | 5 | 5 | 2 | 5 |
| 32 | 2,5 | 5 | 5 | 5 | 5 |
| 33 | 2,5 | 5 | 5 | 2 | 5 |
| 34 | 2,5 | 5 | 5 | 3 | 3 |
| 35 | 2,5 | 4 | 5 | 2 | 3 |
| 36 | 2,5 | 5 | 5 | 3 | 4 |
| 37 | 2,5 | 4 | 5 | 1 | 4 |
| 38 | 2,5 | 5 | 5 | 3 | 5 |
| 39 | 2,5 | 2 | 5 | 1 | 3 |
| 40 | 2,5 | 5 | 5 | 3 | 3 |
| 44 | 2,5 | 5 | 5 | 2 | 4 |
| 47 | 2,5 | 4 | 5 | 2 | 3 |
| 52 | 2,5 | 5 | 5 | 4 | 5 |
| 54 | 2,5 | 4 | 5 | 3 | 3 |
| 57 | 2,5 | 4 | 5 | 2 | 3 |
| 63 | 2,5 | 4 | 5 | 4 | 5 |
| 65 | 2,5 | 5 | 5 | 4 | 4 |

0216243

Fortsetzung Tab. 3

| Bsp.-Nr. | Dosis kg a.i./ha | herbizide Wirkung SIA | CRS | LOM | ECG |
|---|---|---|---|---|---|
| 66 | 2,5 | 5 | 5 | 4 | 4 |
| 69 | 2,5 | 4 | 5 | 2 | 3 |
| 77 | 2,5 | 4 | 5 | 2 | 3 |
| 80 | 2,5 | 4 | 5 | 3 | 5 |
| 82 | 2,5 | 5 | 5 | 3 | 4 |

Abkürzungen:

SIA = Sinapis arvenis
CRS = Chrysanthemum segetum
LOM = Lolium multiflorum
ECG = Echinochloa crus-galli
a.i.= Aktivsubstanz

## 3. Kulturpflanzenverträglichkeit

In weiteren Versuchen im Gewächshaus wurden Samen einer größeren Anzahl von Kulturpflanzen in sandigem Lehmboden ausgelegt und mit Erde abgedeckt.
Ein Teil der Töpfe wurde sofort wie unter 1. beschrieben behandelt; die übrigen wurden im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen

ungeschädigt ließen. Einige Substanzen schonten darüber hinaus auch Gramineen-Kulturen wie z. B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

PATENTANSPRÜCHE:

1. Verbindungen der Formel I

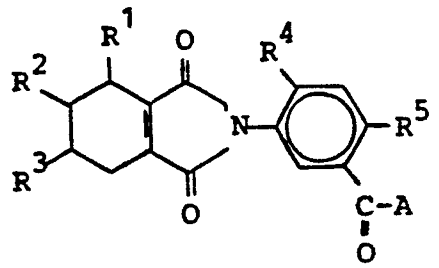

(I),

worin

$R^1$, $R^2$, $R^3$ = unabhängig voneinander Wasserstoff oder $(C_1-C_4)$Alkyl,

$R^4$ = Wasserstoff, F. Cl, Br, $(C_1-C_4)$Alkyl oder $(C_1-C_4)$Alkoxy,

$R^5$ = F, Cl oder Br, $(C_1-C_4)$Alkyl, $(C_1-C_4)$Alkoxy, wobei beide letztgenannten Reste bis zu 3-fach durch Halogen substituiert sein können; Phenoxy, Phenylthio wobei in den beiden letztgenannten Resten der Phenylkern bis zu 3-fach durch Halogen, $(C_1-C_4)$-Alkyl, $(C_1-C_4)$Alkoxy oder $CF_3$ substituiert sein kann, $(C_1-C_4)$Alkylthio, $(C_1-C_4)$Alkylsulfonyl, Phenylsulfonyl, wobei der Phenylring bis zu 3-fach substituiert sein kann durch Halogen, $(C_1-C_4)$Alkyl, $(C_1-C_4)$Alkoxy oder $CF_3$;

A = einen Rest der Formel $-Y\left(\begin{matrix}R_6\\C\\R_6\end{matrix}\right)_n-X-COYR^7$

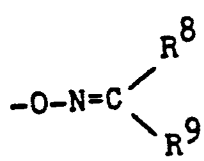 , 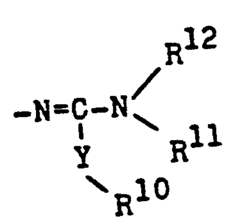 , 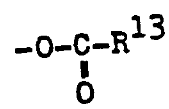

oder 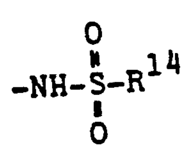 ,

$R^6$ = Wasserstoff, ($C_1$-$C_4$)Alkyl, $CCl_3$, Phenyl, das bis zu 3-fach durch ($C_1$-$C_4$)Alkyl ($C_1$-$C_4$)Alkoxy, Halogen oder $CF_3$ substituiert sein kann; oder ($C_1$-$C_4$)Alkoxycarbonyl($C_1$-$C_4$)-alkyl

$R^7$ = H, ($C_1$-$C_{12}$)Alkyl, ($C_1$-$C_4$)Alkoxy($C_1$-$C_4$)alkyl, ($C_1$-$C_4$)Alkoxycarbonyl($C_1$-$C_4$)alkyl, ($C_2$-$C_4$)-Alkenyl, ($C_3$-$C_4$)Alkinyl, ($C_3$-$C_7$)Cycloalkyl, Halogen($C_1$-$C_4$)alkyl mit ein bis 3 Halogenatomen, Phenyl oder Benzyl, die beide bis zu 3-fach am Phenylring durch Halogen, ($C_1$-$C_4$)-Alkyl, ($C_1$-$C_4$)Alkoxy oder $CF_3$ substituiert sein können,

X = eine direkte Bindung oder einen Rest der Formeln

$-CH_2-$, $-O-CH_2-$ oder $-\overset{\overset{O-}{|}}{C}H(C_1\text{-}C_4\text{Alkyl})$, wobei die beiden letztgenannten Reste jeweils am C-Atom an die Gruppe $-COYR^7$ gebunden sind,

Y = O, S, $NR^{15}$,

$R^8$, $R^9$ = ($C_1$-$C_8$)Alkyl, ($C_3$-$C_7$)Cycloalkyl, ($C_1$-$C_4$)-Alkoxycarbonyl, Phenyl,
Benzyl, die beide im Phenylring 1- bis 3fach durch ($C_1$-$C_4$)-Alkyl, ($C_1$-$C_4$)Alkoxy, das halogeniert sein kann, ($C_1$-$C_4$)Alkylthio, ($C_1$-$C_4$-Alkoxy)-carbonyl, $NO_2$, Halogen, CN oder $CF_3$, substituiert sein können, oder

$R^8$, $R^9$ zusammen mit dem sie verbindenden C-Atom einen 3- bis 7gliedrigen gesättigten oder einfach ungesättigten aliphatischen Ring bilden, der 1- bis 3fach durch ($C_1$-$C_4$)Alkyl, ($C_1$-$C_4$)Alkoxy, Halogen oder ($C_1$-$C_4$-Alkoxy)carbonyl substituiert sein kann,

$R^{10}$ = ($C_1$-$C_4$)Alkyl oder Benzyl,

$R^{11}$, $R^{12}$ = Wasserstoff oder die Gruppe $=CH-N(C_1\text{-}C_4\text{alkyl})_2$

$R^{13}$ = ($C_1$-$C_8$)Alkyl oder Phenyl, das 1- bis 3fach durch Halogen, ($C_1$-$C_4$)-Alkyl, ($C_1$-$C_4$)Alkoxy oder

($C_1$-$C_4$-Alkoxy)carbonyl substituiert sein kann,

$R^{14}$ = Hydroxy oder ($C_1$-$C_4$)Alkyl,

$R^{15}$ = H, ($C_1$-$C_4$)Alkyl oder Phenyl und

n = 1, 2 oder 3

bedeuten.

2. Verbindungen der Formel I von Anspruch 1, worin $R^1$, $R^2$, $R^3$ = Wasserstoff, $R^4$ = H, F oder Cl, $R^5$= F,Cl, Br, ($C_1$-$C_4$)Alkyl oder ($C_1$-$C_4$)Alkoxy, A = einen Rest der Formel 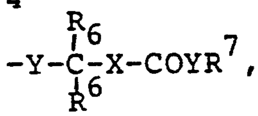, -O-N=$CR^8R^9$ oder 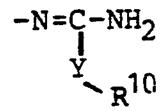

wobei bevorzugt einer der Reste $R^6$ Wasserstoff und der andere ($C_1$-$C_4$)Alkyl oder $CCl_3$ oder beide Reste $R^6$ ($C_1$-$C_4$)-Alkyl bedeuten; $R^7$= ($C_1$-$C_4$)Alkyl, ($C_3$-$C_4$)Alkinyl oder ($C_1$-$C_4$)Alkoxy($C_1$-$C_4$)alkyl; $R^8$,$R^9$= ($C_1$-$C_4$)Alkyl, ($C_1$-$C_4$)-Alkoxycarbonyl oder zusammen mit dem sie verbindenden C-Atom einen ($C_5$-$C_6$)Cycloalkylring bedeuten.

3. Verfahren zur Herstellung von Verbindungen der Formel I gemäß Ansprüchen 1 oder 2, dadurch gekennzeichnet, daß man eine Verbindung der Formel II

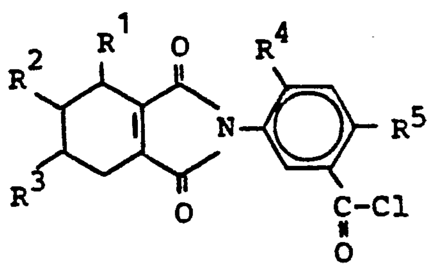

(II)

in Gegenwart einer Base mit einer Verbindung der Formel H-A umsetzt.

4. Verwendung der Verbindungen der Formel I von Ansprüchen 1 oder 2 als Herbizide.

5. Herbizide Mittel, dadurch gekennzeichnet, daß sie eine Verbindung der Formel I von Ansprüchen 1 oder 2 enthalten.

6. Verfahren zur Bekämpfung von unerwünschten Pflanzen, dadurch gekennzeichnet, daß man eine wirksame Menge einer Verbindung der Formel I von Ansprüchen 1 oder 2 auf diese oder die von Ihnen bewachsenen Böden appliziert.

Patentansprüche Österreich



1. Verfahren zur Herstellung von Verbindungen der Formel I

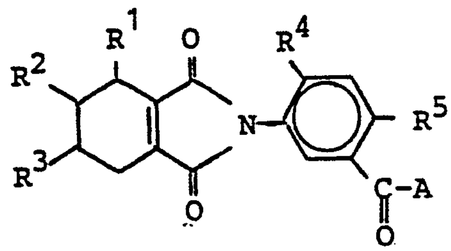

(I),

worin

$R^1$, $R^2$, $R^3$ = unabhängig voneinander Wasserstoff oder ($C_1$-$C_4$)Alkyl,

$R^4$ = Wasserstoff, F, Cl, Br, ($C_1$-$C_4$)Alkyl oder ($C_1$-$C_4$)Alkoxy,

$R^5$ = F, Cl, Br, ($C_1$-$C_4$)Alkyl, ($C_1$-$C_4$)Alkoxy, wobei beide letztgenannten Reste bis zu 3-fach durch Halogen substituiert sein können, Phenoxy, Phenylthio, wobei in den beiden letztgenannten Resten der Phenylkern bis zu 3-fach durch Halogen, ($C_1$-$C_4$)-Alkyl, ($C_1$-$C_4$)Alkoxy oder $CF_3$ substituiert sein kann, ($C_1$-$C_4$)Alkylthio, ($C_1$-$C_4$)Alkylsulfonyl, Phenylsulfonyl, wobei der Phenylring bis zu 3-fach substituiert sein kann durch Halogen, ($C_1$-$C_4$)Alkyl, ($C_1$-$C_4$)Alkoxy oder $CF_3$;

A = einen Rest der Formel 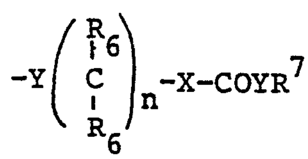

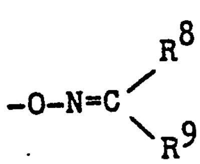

,

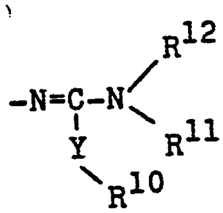

,

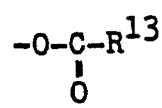

oder 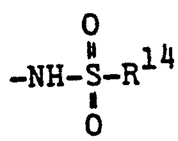

,

$R^6$ = Wasserstoff, $(C_1-C_4)$Alkyl, $CCl_3$, Phenyl, das bis zu 3-fach durch $(C_1-C_4)$Alkyl $(C_1-C_4)$Alkoxy, Halogen oder $CF_3$ substituiert sein kann; oder $(C_1-C_4)$Alkoxycarbonyl$(C_1-C_4)$-alkyl

$R^7$ = H, $(C_1-C_{12})$Alkyl, $(C_1-C_4)$Alkoxy$(C_1-C_4)$alkyl, $(C_1-C_4)$Alkoxycarbonyl$(C_1-C_4)$alkyl, $(C_2-C_4)$-Alkenyl, $(C_3-C_4)$Alkinyl, $(C_3-C_7)$Cycloalkyl, Halogen$(C_1-C_4)$alkyl mit ein bis 3 Halogenatomen, Phenyl oder Benzyl, die beide bis zu 3-fach am Phenylring durch Halogen, $(C_1-C_4)$-Alkyl, $(C_1-C_4)$Alkoxy oder $CF_3$ substituiert sein können,

X = eine direkte Bindung oder einen Rest der Formeln

$-CH_2-$, $-O-CH_2-$ oder $-\overset{O-}{\overset{|}{C}}H(C_1-C_4Alkyl)$, wobei die beiden letztgenannten Reste jeweils am C-Atom an die Gruppe $-COYR^7$ gebunden sind,

Y = O, S, $NR^{15}$,

$R^8$, $R^9$ = $(C_1-C_8)$Alkyl, $(C_3-C_7)$Cycloalkyl, $(C_1-C_4)$-Alkoxycarbonyl, Phenyl,
Benzyl, die beide im Phenylring 1- bis 3fach durch $(C_1-C_4)$-Alkyl, $(C_1-C_4)$Alkoxy, das halogeniert sein kann, $(C_1-C_4)$Alkylthio, $(C_1-C_4$-Alkoxy)-carbonyl, $NO_2$, Halogen, CN oder $CF_3$, substituiert sein können, oder

$R^8$, $R^9$ zusammen mit dem sie verbindenden C-Atom einen 3- bis 7gliedrigen gesättigten oder einfach ungesättigten aliphatischen Ring bilden, der 1- bis 3fach durch $(C_1-C_4)$Alkyl, $(C_1-C_4)$Alkoxy, Halogen oder $(C_1-C_4$-Alkoxy)carbonyl substituiert sein kann,

$R^{10}$ = $(C_1-C_4)$Alkyl oder Benzyl,

$R^{11}$, $R^{12}$ = Wasserstoff oder die Gruppe $=CH-N(C_1-C_4alkyl)_2$

$R^{13}$ = $(C_1-C_8)$Alkyl oder Phenyl, das 1- bis 3fach durch Halogen, $(C_1-C_4)$-Alkyl, $(C_1-C_4)$Alkoxy oder $(C_1-C_4$-Alkoxy)carbonyl substituiert sein kann,

$R^{14}$ = Hydroxy oder $(C_1-C_4)$Alkyl,

$R^{15}$ = H, $(C_1-C_4)$Alkyl oder Phenyl und

n = 1, 2 oder 3

bedeuten,

dadurch gekennzeichnet, daß man eine Verbindung der Formel II

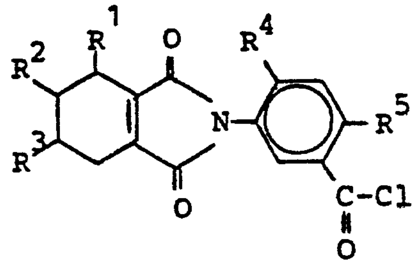

(II)

in Gegenwart einer Base mit einer Verbindung der Formel H-A umsetzt.

2. Verwendung der Verbindungen der Formel I von Anspruch 1 als Herbizide.

3. Herbizide Mittel, dadurch gekennzeichnet, daß sie eine Verbindung der Formel I von Anspruch 1 enthalten.

4. Herbizide Mittel, dadurch gekennzeichnet, daß sie eine Verbindung der Formel I von Anspruch 1 enthalten, worin $R^1$, $R^2$, $R^3$ = Wasserstoff, $R^4$ = H, F oder Cl, $R^5$= F, Cl, Br, $(C_1-C_4)$Alkyl oder $(C_1-C_4)$Alkoxy, A = einen Rest der Formel $-Y-\overset{R^6}{\underset{R^6}{C}}-X-COYR^7$, $-O-N=CR^8R^9$ oder $-N=\underset{Y-R^{10}}{C}-NH_2$

wobei bevorzugt einer der Reste $R^6$ Wasserstoff und der andere $(C_1-C_4)$Alkyl oder $CCl_3$ oder beide Reste $R^6$ $(C_1-C_4)$-Alkyl bedeuten; $R^7$= $(C_1-C_4)$Alkyl, $(C_3-C_4)$Alkinyl oder $(C_1-C_4)$Alkoxy$(C_1-C_4)$alkyl; $R^8$,$R^9$= $(C_1-C_4)$Alkyl, $(C_1-C_4)$-Alkoxycarbonyl oder zusammen mit dem sie verbindenden C-Atom einen $(C_5-C_6)$Cycloalkylring bedeutet.

5. Verfahren zur Bekämpfung von unerwünschtem Pflanzenwuchs in Nutzpflanzenkulturen, dadurch gekennzeichnet, daß man auf die Schadpflanzen oder die Anbaufläche eine Verbindung der Formel I von Ansprüchen 1 oder 4 appliziert.